# EUROPEAN PATENT SPECIFICATION

(11) **EP 2 072 503 B1**
(45) Date of publication and mention of the grant of the patent: **26.10.2011**
(21) Application number: 08170830.7
(22) Date of filing: 05.12.2008
(51) Int. Cl.: C07D 239/52

(54) **Process for the preparation of bosentan**
Verfahren zur Herstellung von Bosentan
Procédé de préparation de bosentan

(30) Priority: 18.12.2007 IT MI20072360; 28.05.2008 IT MI20080992
(43) Date of publication of application: 24.06.2009
(73) Proprietor: Dipharma Francis S.r.l., 20021 Baranzate (MI) (IT)
(72) Inventor: Taddei, Maurizio, 53035 MONTERIGGIONI (SI) (IT); Naldini, Diletta, 50028 TAVARNELLE V.P. (FI) (IT); Allegrini, Pietro, 20097 S. DONATO MILANESE (MI) (IT); Razzetti, Gabriele, 20099 SESTO SAN GIOVANNI (MI) (IT); Mantegazza, Simone, 20144 MILANO (IT)
(74) Representative: Minoja, Fabrizio

(56) References cited:
- EP-A- 0 526 708
- EP-A- 0 743 307
- US-A- 6 136 971

## Description

### FIELD OF THE INVENTION

The present invention relates to a process for the preparation of benzenesulfonamide compounds, in particular 4-tert-butyl-N-[6-(2-hydroxyethoxy)-5-(2-methoxy-phenoxy)-[2,2']bipyrimidinyl-4-yl]-benzene-sulfonamide, namely bosentan, and intermediates useful for its synthesis.

### TECHNOLOGICAL BACKGROUND

Bosentan **(1)** is an endothelin receptor antagonist known from US 5,292,740. which discloses its preparation according to the process schematized below:

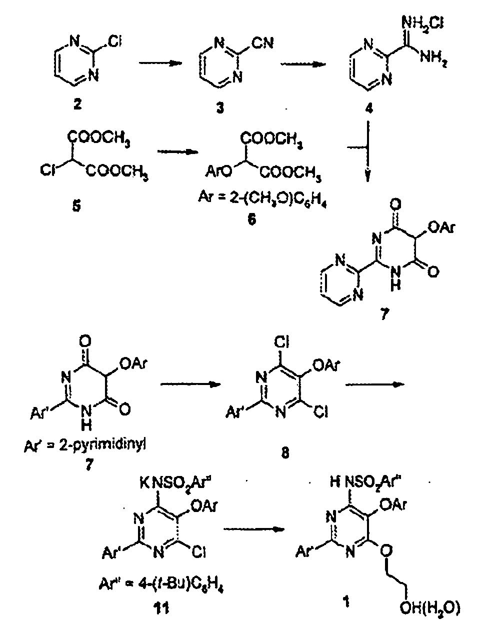

Said process suffers from a number of drawbacks from the industrial point of view. In particular, the last reaction step makes use of ethylene glycol sodium salt, which is a reagent difficult to prepare and use as it is toxic and irritant.

Furthermore, this process involves the formation of impurities: in particular the pyrimidinone **(13)** and the dimer **(12)** depicted hereinbelow.

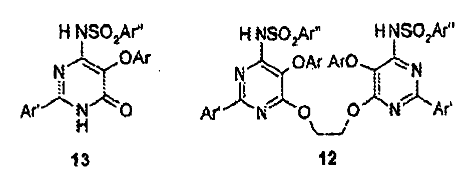

Complex purification processes of the final product are required in order to remove these by-products, thereby involving both operative and economic disadvantages.

Moreover, the last reaction step makes use of a large excess of ethylene glycol which is difficult to remove from the final product, as it is high-boiling, further negatively affecting the process costs.

A further synthetic method is disclosed, for example, in US 6,136,971, according to the process schematized below:

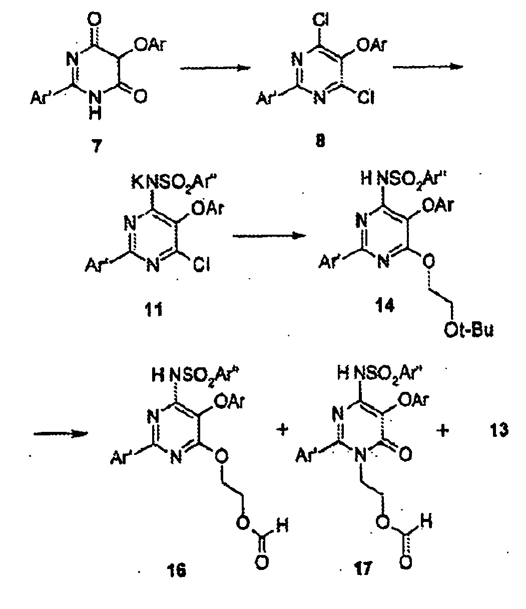

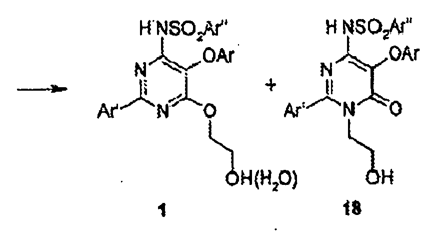

Said synthetic method makes use of the monoprotected ethylene glycol sodium salt, which is more expensive than ethylene glycol and involves the same safety problems as the process described in US 5,292,740. Said method also involves the formation of impurities in the final product, in particular the compounds **(13)** and **(18)** described above.

There is therefore the need for a novel alternative process, which employs inexpensive starting materials and allows to obtain bosentan free from the above mentioned impurities.

### SUMMARY OF THE INVENTION

An alternative process has now been found, which provides bosentan from low cost starting materials, operating under milder conditions than in known methods. More particularly, this process provides bosentan with high purity level. This makes the process of the invention more advantageous and economic than those of the prior art.

### BRIEF DISCLOSURE OF THE ANALYTICAL METHODS

Bosentan crystalline form was characterized by X-Ray Powder Diffraction (XRPD), ¹H-NMR nuclear magnetic resonance spectrometry and Differential Scanning Calorimetry (DSC).

X-ray diffraction spectra (XRPD) were recorded with an APD-2000 automatic diffractometer θ/θ for powders and liquids manufactured by Ital-Structures, under the following operative conditions: CuKα radiation (λ = 1.5418 A), scansion with angular interval 3-40° in 2θ with angular step of 0.03° for 1 sec.

DSC thermograms were recorded with the differential scansion calorimeter Mettler-Toledo DSC 822e, under the following operative conditions: aluminium capsules, 30-300°C interval at the rate of 10°C/min, with nitrogen as purging gas (80 ml/min).

The water content in the compounds was determined by titration according to Karl - Fischer.

Particle size was determined with the known laser light scattering technique using a Malvern Mastersizer MS1 instrumentation under the following operative conditions: a) 300RF mm lens, with 2.4 mm laser beam length; and b) 500 mg sample dispersed in 10 ml hexane (ACS reagent) with 1% SPAN 85^{®}, no presonication, 2500 rpm stirring rate.

### BRIEF DISCLOSURE OF THE FIGURES

Figure 1: XRPD spectrum of bosentan.
Figure 2: DSC thermogram of bosentan.

### DETAILED DISCLOSURE OF THE INVENTION

An object of the invention is a process for the preparation of 4-tert-butyl-N-[6-(2-hydroxy-ethoxy)-5-(2-methoxy-phenoxy)-[2,2']bipyrimidinyl-4-yl]-benzenesulfonamide, of formula **(I),** or a salt or hydrated form thereof, 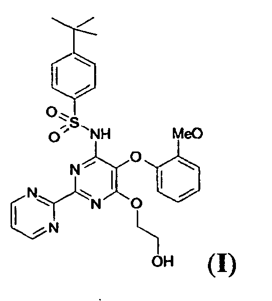 comprising the reaction of a compound of formula (II) or a salt thereof, 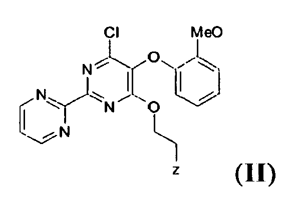 wherein Z is an optionally protected hydroxy group, with a compound of formula **(III)** or a salt thereof, 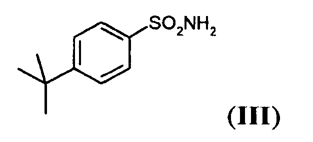 in the presence of a base; and, if necessary, the removal of the hydroxy-protecting group, and/or, if desired, the conversion of a compound of formula **(I)** to a salt thereof, or *vice versa.*

A hydroxy-protecting group can be for example one of the protective groups used in the alcohols chemistry, typically an acyl group, e.g. a C₁-C₆ alkanoyl group, preferably a C₁-C₄ alkanoyl group, in particular formyl, acetyl or propionyl; an aryl-C₁-C₆ alkanoyl group, e.g. phenylacetyl, phenylpropionyl, or aroyl, e.g. benzoyl, wherein the phenol ring is optionally substituted with one to three substituents independently selected e.g. from halogen, in particular chlorine, bromine or iodine, and cyano; an aryl-C₁-C₆ alkyl group, e.g. benzyl, phenylethyl or naphthalenylmethyl; or a tri (C₁-C₆) alkyl-silyl group, e.g. trimethylsilyl, tert-butyl-dimethylsilyl. Preferably a C₁-C₆ alkanoyl group, more preferably a C₁-C₄ alkanoyl group, in particular formyl or acetyl.

A base can be an organic base, for example an alkali metal C₁-C₆ alkoxide, such as sodium or potassium methoxide, ethoxide or tert-butoxide; sodium hydride, 1,8-Diazabicyclo[5.4.0]undec-7-ene (DBU), 1,4-diazabicyclo[2.2.2]octane (DABCO); or an inorganic base, e.g. an alkali or alkaline-earth metal hydroxide, carbonate or phosphate, e.g. sodium, potassium or barium hydroxide, sodium or potassium carbonate, or sodium or potassium phosphate. The base is preferably an inorganic base, more preferably an alkali or alkaline-earth metal phosphate; in particular potassium or sodium phosphate.

The molar ratio of a compound of formula **(III)** to a compound of formula **(II)** can approximately range from 1 to 2; preferably approximately from 1 to 1.5; in particular around 1.2.

The molar ratio of the base to a compound of formula **(II)** can approximately range from 1 to 10; preferably approximately from 2 to 5; in particular around 3.

Optionally, the reaction can be carried out in the presence of a catalyst, and, if necessary, of a ligand.

A catalyst can for example be based on a transition metal, typically copper. The catalyst is typically used as a salt; preferred examples are copper (I) salts, such as copper (I) iodide, chloride, bromide, ortho-triflate or acetate; preferably copper (I) iodide.

When the catalyst is used in the presence of a ligand, this can be an organic ligand, typically an amino acid, selected from e.g. glycine, cysteine, lysine, α-alanine, β-alanine. The ligand is preferably an amino acid; typically glycine or α-alanine, in particular glycine.

The molar ratio of the catalyst to a compound of formula **(II)** can approximately range from 0.01 to 0.5; preferably approximately from 0.03 to 0.2; in particular around 0.04 - 0.1.

The reaction, independently of the use of a catalyst, can be carried out in a solvent, typically an organic solvent, selected from e.g. a dipolar aprotic solvent, typically dimethylformamide, dimethylacetamide, acetonitrile, dimethylsulfoxide; an ether, e.g. diethyl ether, methyl tert-butyl ether, tetrahydrofuran or dioxane; a chlorinated solvent, e.g., dichloromethane, chloroform or chlorobenzene; an apolar solvent, such as an aliphatic hydrocarbon, e.g. hexane or cyclohexane, or an aromatic hydrocarbon, e.g. benzene or toluene; an ester, e.g. ethyl or methyl acetate; a ketone, e.g. acetone, methyl ethyl ketone, methyl isobutyl ketone; a C₁-C₆ alkanol, e.g. methanol, ethanol, isopropanol or tert-butanol; or a mixture of two or more, preferably of two or three, of said solvents. Alternatively, the reaction can be carried out in water or mixtures of water with one or more, preferably one or two, of said solvents. The reaction is preferably carried out in the presence of an organic dipolar aprotic solvent, more preferably dimethylformamide or dimethylacetamide, in particular dimethylacetamide.

The reaction can be carried out at a temperature ranging from 0°C to the reflux temperature of the reaction mixture, for example using dimethylacetamide as the solvent. The reaction can be carried out at a temperature of about 65°C.

The removal of the hydroxy-protecting group can be effected according to methods known in the art, for example by hydrolysis.

A compound of formula **(I)** can be converted to a salt thereof, or *vice versa,* according to known methods.

A compound of formula **(II)** as defined above, and the salts thereof, are novel and are a further object of the invention.

Preferred examples compounds of formula **(II)** are:
- 2-(5-(2-methoxy-phenoxy)-6-chloro-2-(pyrimidin-2-yl)pyrimidin-4-yloxy) ethanol formyl ester;
- 2-(5-(2-methoxy-phenoxy)-6-chloro-2-(pyrimidin-2-yl)pyrimidin-4-yloxy) ethanol acetyl ester;
- 2-(5-(2-methoxy-phenoxy)-6-chloro-2-(pyrimidin-2-yl)pyrimidin-4-yloxy) ethanol benzoyl ester;

- 2-(5-(2-methoxy-phenoxy)-6-chloro-2-(pyrimidin-2-yl)pyrimidin-4-yloxy) ethanol trimethylsilyl ether;
- 2-(5-(2-methoxy-phenoxy)-6-chloro-2-(pyrimidin-2-yl)pyrimidin-4-yloxy) ethanol tert-butyl-dimethylsilyl ether;
- 2-(5-(2-methoxy-phenoxy)-6-chloro-2-(pyrimidin-2-yl)pyrimidin-4-yloxy) ethanol benzyl ether;
- 2-(5-(2-methoxy-phenoxy)-6-chloro-2-(pyrimidin-2-yl)pyrimidin-4-yloxy) ethanol phenylethyl ether; and
- 2-(5-(2-methoxy-phenoxy)-6-chloro-2-(pyrimidin-2-yl)pyrimidin-4-yloxy) ethanol naphthalenylmethyl ether.

A compound of formula **(II)** wherein Z is a hydroxy group can be obtained by a process comprising the reaction of a compound of formula **(IV)** or a salt thereof, 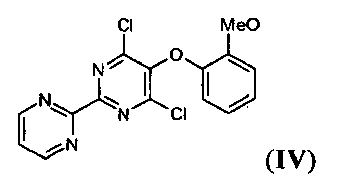 with ethylene glycol, in the presence of a base.

A base is typically an organic base, in particular a tertiary amine, for example a tri(C₁-C₆)alkylamine, e.g. triethylamine or trimethylamine, a tri(C₁-C₆)alkanolamine, e.g. triethanolamine, trimethanolamine or tripropanolamine, or diazabicyclooctane or diazabicycloundecene, or mixtures of two or more, preferably of two or three of said bases. The base is preferably a tri(C₁-C₆)alkanolamine, in particular triethanolamine.

The reaction can be carried out in a solvent, which can be an excess of ethylene glycol itself, typically about 3-10 volumes, preferably about 5-6 volumes, of ethylene glycol per volume of substrate, or an organic solvent, selected from e.g. those mentioned above for the reaction of a compound of formula **(II)** with a compound of formula **(III),** except for those which contain reactive groups known to be liable to react with the compound of formula **(IV),** for example alcohols; or a mixture of two or more, preferably of two or three, of said solvents. The solvent is preferably a dipolar aprotic solvent, in particular acetonitrile.

When the reaction is carried out in a solvent, the molar ratio of ethylene glycol to a compound of formula **(IV)** can approximately range from 1 to 5; preferably approximately from 1 and 2; in particular around 1.7.

The reaction can be carried out at a temperature ranging from 0°C to the reflux temperature of the reaction mixture, preferably at the reflux temperature of the reaction mixture.

A compound of formula **(II)** wherein Z is a protected hydroxy group can be prepared from a compound of formula **(II),** wherein Z is a free hydroxy group, according to known methods.

A compound of formula **(IV)** is known, and can be prepared according to known methods, for example as disclosed in US 5,292,740.

It has now surprisingly been found that the reaction between a compound of formula **(IV)** and ethylene glycol can be carried out under much milder conditions than those of the prior art, as far as compound **(11)** is concerned, thereby dramatically reducing the formation of by-products and any dimers of the compound of formula **(II),** analogous to compound **(12).**

If desired, a compound of formula **(II)** can be easily purified to remove by-products and any impurities formed during the reaction, according to known methods, for example by chromatography. The purification of said intermediate is more convenient than the purification of the final product from the industrial point of view.

A resulting compound of formula **(II)** has a purity equal to or higher than 99.5%, preferably equal to or higher than 99.9%.

Bosentan, of formula **(I),** as obtainable starting from a compound of formula **(II)** having such purity characteristics, has purity equal to or higher than the starting compound, i.e. equal to or higher than 99.5%, preferably equal to or higher than 99.9%.

Bosentan, as obtainable according to the process of the invention, has a mean particle size D₅₀ approximately ranging from 5 to 250 micrometers, preferably approximately from 10 to 100 micrometers. Said size can be further reduced by a fine grinding process following known techniques or it can be increased by controlling the crystallization conditions, for example by slowly cooling the solution, as it is known in the art.

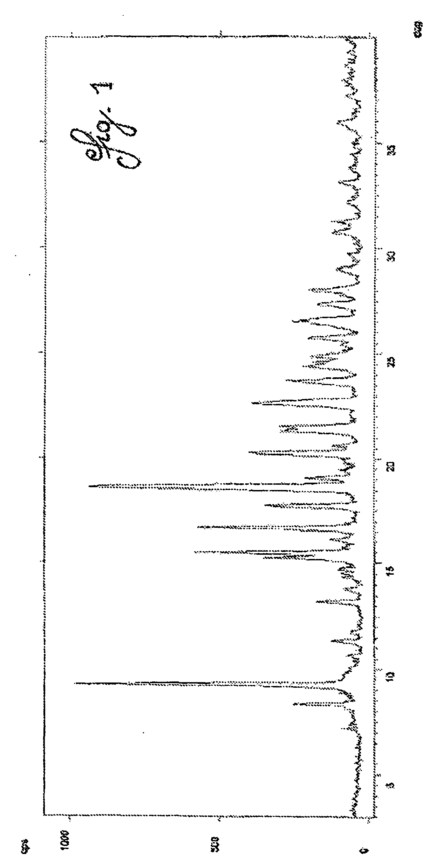

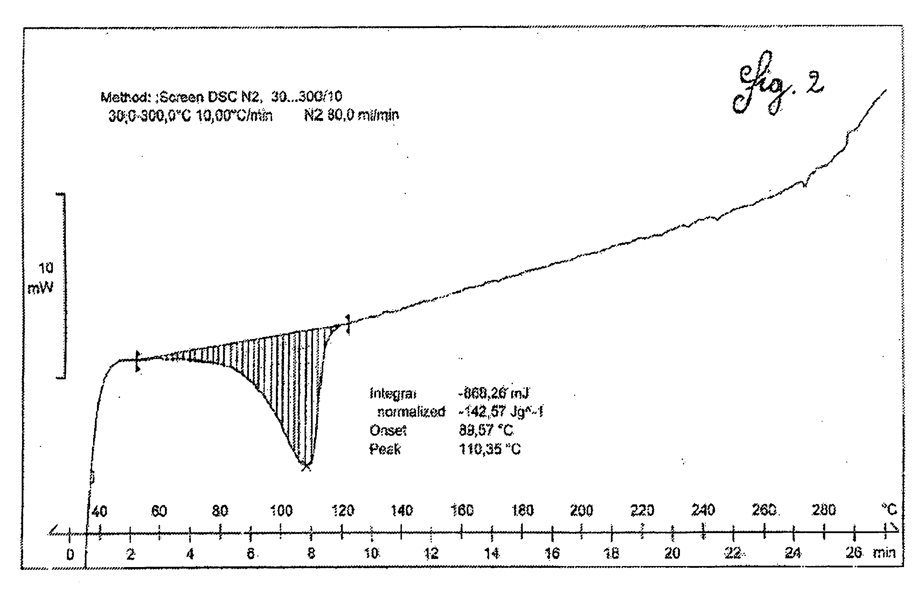

Bosentan, according to the process of the invention, is in a crystalline form, having an XRPD spectrum as reported in Figure 1, wherein the most intense diffraction peaks are expressed in 2θ ± 0.2°; a DSC thermogram as reported in Figure 2, with an exothermic peak at 110° ± 2°C; and a water content approximately ranging from 2.5 to 3.5%, thus it can be defined as substantially monohydrate.

**Table**

| 2*θ* ± 0.2 2*θ* | I/Iₘₐₓ |
|---|---|
| 8.34 | 27 |
| 9.24 | 94 |
| 15.24 | 38 |
| 15.51 | 62 |
| 16.68 | 59 |
| 17.73 | 36 |
| 18.63 | 100 |
| 20.25 | 41 |
| 21.33 | 30 |
| 22.65 | 41 |

These characteristics are substantially the same as those of bosentan obtained strictly following the procedures of the experimental Example 8 of US 6,136,971.

In the present invention, compound of formula **(I), (II), (III)** and **(IV)** means the compound as it is or a salt thereof, in particular a pharmaceutically acceptable salt thereof with an acid or a base selected from those commonly used in the art; for example sulfate, hydrochloride, acetate, formate, propionate, or sodium, potassium, ammonium salts. Said compounds can be converted to the salts thereof, or *vice versa*, according to known methods.

The following examples illustrate the invention.

### Example 1: 4-tert-Butyl-N-[6-(2-hydroxy-ethoxy)-5-(2-methoxy-phenoxy)-[2,2']bipyrimidinyl-4-yl]-benzenesulfonamide; (bosentan), (I)

A 10 ml round-bottom flask is loaded with CuI (2.47 mg, 5 mol %), 4-tert-butylbenzenesulfonamide (0.07 g, 0.31 mmol), glycine (3.9 mg, 20 mol %) and K₃PO₄ (0.14 g, 0.65 mmol). Three vacuum/nitrogen cycles are performed, then compound of formula **(II)** (0.10 g, 0.26 mmol) and dry dioxane (1 ml) are added under nitrogen. The round-bottom flask is closed with a screw cap, and the mixture is kept at about 120°C under strong stirring for about 44 hours. The suspension is filtered through Celite and the residue is taken up in ethyl acetate. The filtrate is concentrated and the residue purified by flash chromatography eluting with CHCl₃/MeOH 99:1. 0.05 g of the title product are obtained (yield: 31%).
¹H-NMR (CDCl₃, 200 MHz, 25°C) δ: 8.98-8.96 (d, *J*=*4.8 Hz*, 2H, Ar), 8.38 (br d, 2H, S-Ph), 7.43 (d, *J*=8.3, 2H, S-Ph), 7.36 (m, 1H, Ar), 7.24-6.99 (m, 4H, Ph, S-Ph), 4.58 (t, *J=*7.7 Hz, 2H, CH₂), 3.91 (s, 3H, OCH₃), 3.87-3.81 (m, 2H, CH₂OH), 1.27 (s, 9H, C(CH)₃).
ES/MS: C₂₇H₂₉N₅O₆S, 552 [M+H]⁺, 574 [M+Na]⁺, 1125 [2M+Na]⁺.

The resulting compound has approximately 99.5% purity and mean particle size D₅₀ around 50 micrometers.

### Example 2: 2-(5-(2-Methoxy-phenoxy)-6-chloro-2-(pyrimidin-2-yl)pyrimidin-4-yloxy) ethanol; (II)

A solution of freshly distilled triethanolamine (0.17 g, 1.72 mmol) in dry ethylene glycol (8 ml) is added with a solution of 4,6-dichloro-5-(2-methoxyphenoxy)pyrimidine (0.30 g, 9086 mmol) in acetonitrile (4.5 ml). The mixture is refluxed for 12 hours, the solvent is evaporated off under vacuum and the remaining solution is acidified with 1M hydrochloric acid solution and extracted with ethyl acetate. The combined organic phases are washed with water and a sodium chloride saturated solution; then dried over sodium sulfate. The solvent is evaporated off under reduced pressure and the residue is purified by flash chromatography eluting with CHCl₃/MeOH 99:1. 0.30 g of a white solid are obtained (m.p. 162-164°C), in 93% yield. The resulting compound has approximately 99.7% purity.
¹H-NMR (CDCl₃, 400 MHz, 25°C) δ: 8.97-8.96 (d, *J=4.8 Hz*, 2H, Ar), 7.39 (t, *J=4.8 Hz*, 1H, Ar), 7.09-7.05 (m, 1H, Ph), 6.98-6.96 (m, 1H, Ar), 6.87-6.83 (m, 1H, Ph), 6.78-6.76 (m, 1H, Ph), 4.58 (t, *J=7.7 Hz*, 2H, CH₂), 3.91 (s, 3H, OCH₃), 3.85-3.79 (m, 2H, CH₂OH).
¹³C NMR (CDCl₃, 100 MHz, 25°C) δ: 162.558, 160.755, 157.999, 155.319, 152.993, 149.484, 145.564, 135.590, 124.528, 121.550, 120.854, 116.510, 112.934, 72.078, 62.042, 56.297.
ES/MS: C₁₇H₁₅ClN₄O₄, 375 [M+H]⁺, 397 [M+Na]⁺, 413 [M+K]⁺, 771 [2M+23]⁺.

### Example 3 - Synthesis of 2-(5-(2-Methoxy-phenoxy)-6-chloro-2-(pyrimidin-2-yl)pyrimidin-4-yloxy) ethanol; (II)

A round-bottom flask under nitrogen atmosphere is loaded with 4,6-dichloro-5-(2-methoxy-phenoxy)-[2,2']bipyrimidinyl (250 g, 0.716 mol) and triethylamine (145 g, 1.43 mol) suspended in ethylene glycol (1250 ml) and the mixture is heated at about 100°C for 3-4 hours. The reaction mixture is diluted with water (1250 ml) and cooled at 0-5°C in about 2 hours. The solid is filtered and washed with cold water (3 x 250 ml), then with isopropanol (250 ml). The product is dried in a static dryer under vacuum at about 50°C for 18 hours, to obtain 260 g of the title product, in 96.7% yield.

### Example 4 - Synthesis of 4-tert-Butyl-N-[6-(2-hydroxy-ethoxy)-5-(2-methoxy-phenoxy)-[2,2']bipyrimidinyl-4-yl]-benzenesulfonamide; (bosentan)

A round-bottom flask under nitrogen at room temperature is loaded with 4-tert-butyl-benzenesulfonamide (14.0 g, 0.064 mol), tribasic potassium phosphate (28.1 g, 0.132 mol) and 2-(5-(2-methoxy-phenoxy)-6-chloro-2-(pyrimidin-2-yl)pyrimidin-4-yloxy) ethanol (20.0 g, 0.053 mol) suspended in N,N-dimethyl-acetamide (100 ml) and the reaction mixture is heated at 100°C for 18 hours. The reaction mixture is diluted with water (200 ml), acidified to pH 4-5 with 37% HCl and extracted with toluene (3 x 100 ml). Bosentan is obtained by chromatographic purification.

### Example 5 - Synthesis of 2-(5-(2-Methoxy-phenoxy)-6-chloro-2-(pyrimidin-2-yl)pyrimidin-4-yloxy) ethanol acetyl ester; (II)

A round-bottom flask under nitrogen atmosphere is loaded with 2-(5-(2-methoxy-phenoxy)-6-chloro-2-(pyrimidin-2-yl)pyrimidin-4-yloxy)ethanol 112 g, 0.300 mol), toluene (450 ml) and the suspension is added with acetic anhydride (45.8 g, 0.450 mol) and triethylamine (45.3 g, 0.450 mol). The mixture is heated to about 95-100°C and reacted for 3-4 hours. The final solution is diluted with water (450 ml) and cooled at about 0-5°C for at least 30 minutes. The product is filtered, washing the solid with cold water (3 x 100 ml) and then with toluene (100 ml). The solid is dried in a static dryer at 50°C for 16 hours. 122 g of product are obtained in 97.5% yield.
¹H-NMR (CDCl₃, 300 MHz) δ: 8.95 (d, 2H); 7.40 (t, 1H); 7.05 (t, 1H); 6.95 (d, 1H); 6.80 (t, 1H), 6.75 (d, 1H); 4.70 (m, 2H); 4.20 (m, 2H); 3.85 (s, 3H); 1.90 (s, 3H).

According to a similar procedure, the following compounds are obtained:
- 2-(5-(2-methoxy-phenoxy)-6-chloro-2-(pyrimidin-2-yl)pyrimidin-4-yloxy) ethanol formyl ester;
- 2-(5-(2-methoxy-phenoxy)-6-chloro-2-(pyrimidin-2-yl)pyrimidin-4-yloxy) ethanol benzoyl ester;
- 2-(5-(2-methoxy-phenoxy)-6-chloro-2-(pyrimidin-2-yl)pyrimidin-4-yloxy) ethanol trimethylsilyl ether;
- 2-(5-(2-methoxy-phenoxy)-6-chloro-2-(pyrimidin-2-yl)pyrimidin-4-yloxy) ethanol tert-butyl-dimethylsilyl ether;
- 2-(5-(2-methoxy-phenoxy)-6-chloro-2-(pyrimidin-2-yl)pyrimidin-4-yloxy) ethanol benzyl ether;
- 2-(5-(2-methoxy-phenoxy)-6-chloro-2-(pyrimidin-2-yl)pyrimidin-4-yloxy) ethanol phenylethyl ether; and
- 2-(5-(2-methoxy-phenoxy)-6-chloro-2-(pyrimidin-2-yl)pyrimidin-4-yloxy) ethanol naphthalenylmethyl ether.

### Example 6 - Synthesis of 2-[6-(4-tert-Butyl-benzenesulfonylamino)-5-(2-methoxy-phenoxy)-[2,2']bipyrimidinyl-4-yloxy]-ethanol acetyl ester

In a 3000 ml round-bottom flask under nitrogen atmosphere, 4-tert-butyl-benzenesulfonamide (103 g, 0.480 mol) and tribasic potassium phosphate (255 g, 1.200 mol) are suspended in N,N-dimethyl-acetamide (500 ml) and the mixture is heated at 75-80°C for about 30 minutes. The reaction mixture is cooled to about 65-70°C and added with 2-(5-(2-methoxy-phenoxy)-6-chloro-2-(pyrimidin-2-yl)pyrimidin-4-yloxy) ethanol acetyl ester (200 g, 0.480 mol). The mixture is reacted for 96 hours then diluted with water (1500 ml) and cooled at 0-5°C for at least 1 hour. The solid is filtered, washing with cold water (4 x 250 ml), and dried at about 45-50°C for 16-18 hours. 262 g of a solid crude are obtained, in 92% yield. The resulting product is used for the next reaction, as described in Example 7.
¹H-NMR (CDCl₃, 300 MHz) δ: 9.00 (d, 2H); 8.84 (s, 1H, exch. with D₂O); 8,40 (d, 2H); 7.44-7.36 (m, 3H); 7.20-7.06 (m, 2H); 9.96 (d, 1H); 6.84 (t, 1H); 4.70 (m, 2H); 4.28 (m, 2H); 3.92 (s, 3H); 1.90 (s, 3H); 1.38 (s, 9H).

### Example 7 - Synthesis of 4-tert-Butyl-N-[6-(2-hydroxy-ethoxy)-5-(2-methoxy-phenoxy)-[2,2']bipyrimidinyl-4-yl]-benzenesulfonamide; (Bosentan sodium salt)

The solid from Example 6 (245 g) is suspended in ethanol (600 ml) and water (170 ml), then a 30% sodium hydroxide solution (165 g, 1.24 mol) is added and the mixture is reacted at room temperature for about 2-3 hours. The suspended solid is filtered and washed with an ethanol/water 9:1 mixture (2 x 150 ml) then with only ethanol (150 ml). The product is dried in a static dryer a 45-50°C for about 16 hours, to obtain a white solid, 238 g, in 98% yield.
¹H-NMR (DMSO, 300 MHz) δ: 8,9 (d, 2H); 7,7 (d, 2H), 7,6 (t, 1H); 7,3 (d, 2H); 7.0 (d, 1H); 6,9 (t, 1H); 6,7 (t, 1H); 6,4 (d, 1H); 4,3 (m, 2H); 3,8 (s, 3H); 3,6 (m, 2H); 1,2 (s, 9H).

### Example 8 - 4-tert-Butyl-N-[6-(2-hydroxy-ethoxy)-5-(2-methoxy-phenoxy)-[2,2']bipyrimidinyl-4-yl]-benzenesulfonamide (Bosentan)

A 2000 ml round-bottom flask is loaded with 4-tert-butyl-N-[6-(2-hydroxy-ethoxy)-5-(2-methoxy-phenoxy)-[2,2']bipyrimidinyl-4-yl]-benzenesulfonamide sodium salt (200 g) in ethanol (500 ml), the mixture is heated to the reflux temperature and water is added (150 ml) until dissolution of the product. The solution is slowly cooled at 50-55°C, a product crystallizes, and the temperature is kept for about 1h. Then temperature is cooled to 15-20°C and the solid is filtered, washing with an ethanol/water 9:1 mixture (100 ml), then with ethanol (100 ml). The product is suspended in ethanol (500 ml) and slowly acidified with 37% HCl (35 g) to pH 2-3 keeping the temperature below 30°C. The mixture is slowly diluted with water (475 ml) and left under stirring at room temperature for about 3 hours. The solid is filtered and washed with an ethanol/water 1:1 mixture (2 x 75 ml), the product is dried at 25-30°C under reduced pressure. 183 g of product are obtained, in 95% yield. The resulting product has approximately 99.9% purity, mean particle size D₅₀ around 15 micrometers and is in a crystalline form, having the X ray diffraction spectrum as reported in Figure 1, wherein the most intense diffraction peaks fall at 8.34; 9.24; 15.24; 15.51; 16.68; 17.73; 18.63; 20.25; 21.33 and 22.65 ± 0.2° in 2θ; the DSC thermogram as reported in Figure 2, with an exothermic peak at 110° ± 2°C; and a water content approximately ranging from 2.5 to 3.5%.

### Example 9 - Synthesis of 2-[6-(4-tert-Butyl-benzenesulfonylamino)-5-(2-methoxy-phenoxy)-[2,2']bipyrimidinyl-4-yloxy]-ethanol acetyl ester

In a 3000 ml round-bottom flask under nitrogen atmosphere, 4-tert-butyl-benzenesulfonamide (103 g, 0.480 mol) and potassium carbonate (166 g, 1.200 mol) are suspended in N,N-dimethyl-acetamide (300 ml) and acetonitrile (200 ml) and the mixture is heated at 75-80°C for about 30 minutes. The reaction mixture is cooled to about 65-70°C and added with 2-(5-(2-methoxy-phenoxy)-6-chloro-2-(pyrimidin-2-yl)pyrimidin-4-yloxy) ethanol acetyl ester (200 g, 0.480 mol). The mixture is reacted for 96 hours then diluted with water (1500 ml) and cooled at 0-5°C for at least 1 hour. The solid is filtered, washing with cold water (4 x 250 ml), and dried at about 45-50°C for 16-18 hours. 213 g of a solid crude are obtained, in 75% yield. The resulting product is used for the next reaction, as described in Example 7.

### Example 10 - Synthesis of 2-[6-(4-tert-Butyl-benzenesulfonylamino)-5-(2-methoxy-phenoxy)-[2,2']bipyrimidinyl-4-yloxy]-ethanol acetyl ester

In a 3000 ml round-bottom flask under nitrogen atmosphere, 4-tert-butyl-benzenesulfonamide (103 g, 0.480 mol) and potassium carbonate (331 g, 2.400 mol) are suspended in dimethylsulfoxide (500 ml) and the mixture is heated at 75-80°C for about 30 minutes. The reaction mixture is cooled to about 65-70°C and added with 2-(5-(2-methoxy-phenoxy)-6-chloro-2-(pyrimidin-2-yl)pyrimidin-4-yloxy) ethanol acetyl ester (200 g, 0.480 mol). The mixture is reacted for 96 hours then diluted with water (1500 ml) and cooled at 0-5°C for at least 1 hour. The solid is filtered, washing with cold water (4 x 250 ml), and dried at about 45-50°C for 16-18 hours. 171 g of a solid crude are obtained, in 60% yield. The resulting product is used for the next reaction, as described in Example 7.

## Claims

1. A process for the preparation of 4-tert-butyl-N-[6-(2-hydroxy-ethoxy)-5-(2-methoxy-phenoxy)-[2,2']bipyrimidinyl-4-yl]-benzenesulfonamide, of formula **(I),** or a salt or hydrated form thereof, 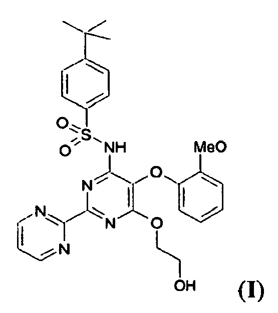 comprising the reaction of a compound of formula **(II),** or a salt thereof, 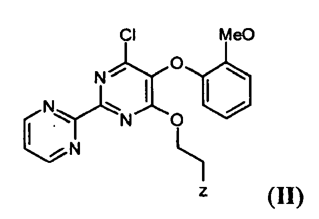 wherein Z is an optionally protected hydroxy group,
with a compound of formula **(III)** or a salt thereof, 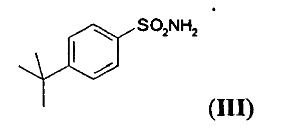 in the presence of a base; and, if necessary, the removal of the hydroxy-protecting group, and/or, if desired, the conversion of a compound of formula **(I)** to a salt thereof, or *vice versa.*

2. A process according to claim 1, wherein the protective group is selected from an acyl group; an aryl C₁-C₆ alkyl group; and a tri (C₁-C₆) alkyl-silyl group.

3. A process according to claim 1, wherein the base is selected from an alkali metal C₁-C₆ alkoxide; an alkali or alkaline-earth metal hydroxide, carbonate or phosphate.

4. A process according to claim 2, wherein the base is an alkali or alkaline-earth metal phosphate.

5. A process according to claim 1, wherein the molar ratio of a compound of formula **(III)** to a compound of formula **(II)** approximately ranges from 1 to 2.

6. A process according to claim 1, wherein the molar ratio of the base to a compound of formula **(II)** approximately ranges from 1 to 5.

7. A process according to claim 1, wherein the reaction is carried out in the presence of a catalyst, and, if necessary of a ligand.

8. A compound of formula **(II)** or a salt thereof, 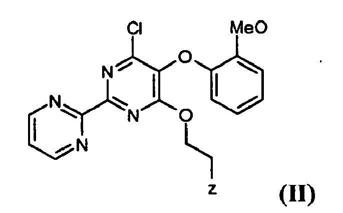 wherein Z is protected hydroxy group.

9. A compound of formula (II) according to claim 8, which is:
• 2-(5-(2-methoxy-phenoxy)-6-chloro-2-(pyrimidin-2-yl)pyrimidin-4-yloxy) ethanol formyl ester;
• 2-(5-(2-methoxy-phenoxy)-6-chloro-2-(pyrimidin-2-yl)pyrimidin-4-yloxy) ethanol acetyl ester;
• 2-(5-(2-methoxy-phenoxy)-6-chloro-2-(pyrimidin-2-yl)pyrimidin-4-yloxy) ethanol benzoyl ester;
• 2-(5-(2-methoxy-phenoxy)-6-chloro-2-(pyrimidin-2-yl)pyrimidin-4-yloxy) ethanol trimethylsilyl ether;
• 2-(5-(2-methoxy-phenoxy)-6-chloro-2-(pyrimidin-2-yl)pyrimidin-4-yloxy) ethanol tert-butyl-dimethylsilyl ether;
• 2-(5-(2-methoxy-phenoxy)-6-chloro-2-(pyrimidin-2-yl)pyrimidin-4-yloxy) ethanol benzyl ether;
• 2-(5-(2-methoxy-phenoxy)-6-chloro-2-(pyrimidin-2-yl)pyrimidin-4-yloxy) ethanol phenylethyl ether; or
• 2-(5-(2-methoxy-phenoxy)-6-chloro-2-(pyrimidin-2-yl)pyrimidin-4-yloxy) ethanol naphthalenylmethyl ether.

10. A process according to claim 1, wherein a compound of formula **(II),** or a salt thereof, is obtained by a process comprising the reaction of a compound of formula **(IV)** 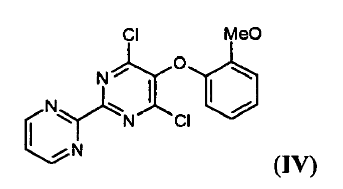 with ethylene glycol, in the presence of a base.

11. A process according to claim 10, wherein the base is selected from a tri(C₁-C₆)alkylamine, diazabicyclooctane and diazabicycloundecene or mixtures thereof.

12. A process according to claim 10, wherein the reaction is carried out in the presence of a ethylene glycol excess.

13. A process according to claim 10, wherein the reaction is carried out in the presence of an organic solvent, and the molar ratio of ethylene glycol to a compound of formula **(IV)** approximately ranges from 1 to 5.

## Patentansprüche

1. Verfahren für die Herstellung von 4-tert-Butyl-N-[6-(2-hydroxyethoxy)-5-(2-methoxyphenoxy)-[2,2']bipyrimidinyl-4-yl]-benzolsulfonamid der Formel (I) oder eines Salzes oder einer hydratisierten Form davon, 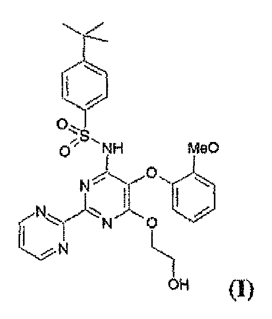 umfassend die Reaktion einer Verbindung der Formel **(II),** oder eines Salzes davon, 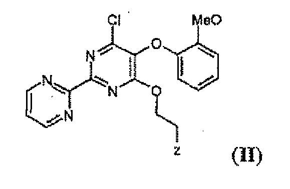 wobei Z eine wahlweise geschützte Hydroxygruppe mit einer Verbindung der Formel **(III)** oder eines Salzes davon ist, 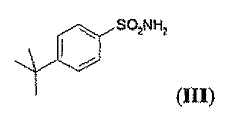 in der Anwesenheit einer Base; und, falls erforderlich, die Entfernung der Hydroxyschutzgruppe und/oder, falls gewünscht, die Umsetzung einer Verbindung der Formel **(I)** in ein Salz davon oder umgekehrt.

2. Verfahren nach Anspruch 1, wobei die Schutzgruppe ausgewählt wird aus einer Acylgruppe; einer Aryl-C₁-C₆-Alkylgruppe und einer Tri-(C₁-C₆)-Alkylsilylgruppe.

3. Verfahren nach Anspruch 1, wobei die Base ausgewählt wird aus einem Alkalimetall-C₁-C₆-Alkoxid; einem Alkali- oder Erdalkalimetallhydroxid, -carbonat oder -phosphat.

4. Verfahren nach Anspruch 2, wobei die Base ein Alkali- oder Erdalkalimetallphosphat ist.

5. Verfahren nach Anspruch 1, wobei das Molverhältnis einer Verbindung der Formel **(III)** zu einer Verbindung der Formel **(II)** ungefähr von 1 bis 2 reicht.

6. Verfahren nach Anspruch 1, wobei das Molverhältnis der Base zu einer Verbindung der Formel **(II)** ungefähr von 1 bis 5 reicht.

7. Verfahren nach Anspruch 1, wobei die Reaktion in Anwesenheit eines Katalysators und, wenn notwendig, eines Liganden durchgeführt wird.

8. Verbindung der Formel **(II)** oder eines Salzes davon, 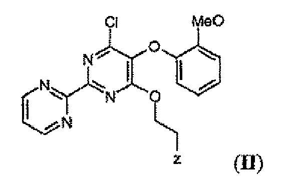 wobei Z eine geschützte Hydroxygruppe ist.

9. Verbindung der Formel **(II)** nach Anspruch 8, welche ist:
• 2-(5-(2-Methoxyphenoxy)-6-chlor-2-(pyrimidin-2-yl)pyrimidin-4-yloxy)ethanolformylester;
• 2-(5-(2-Methoxyphenoxy)-6-chlor-2-(pyrimidin-2-yl)pyrimidin-4-yloxy)ethanolacetylester;
• 2-(5-(2-Methoxyphenoxy)-6-chlor-2-(pyrimidin-2-yl)pyrimidin-4-yloxy)ethanolbenzoylester;
• 2-(5-(2-Methoxyphenoxy)-6-chlor-2-(pyrimidin-2-yl)pyrimidin-4-yloxy)ethanoltrimethylsilylether;
• 2-(5-(2-Methoxyphenoxy)-6-chlor-2-(pyrimidin-2-yl)pyrimidin-4-yloxy)ethanol-tert-butyldimethylsilylether;
• 2-(5-(2-Methoxyphenoxy)-6-chlor-2-(pyrimidin-2-yl)pyrimidin-4-yloxy)ethanolbenzylether;
• 2-(5-(2-Methoxyphenoxy)-6-chlor-2-(pyrimidin-2-yl)pyrimidin-4-yloxy)ethanolphenylethylether; oder
• 2-(5-(2-Methoxyphenoxy)-6-chlor-2-(pyrimidin-2-yl)pyrimidin-4-yloxy)ethanolnaphthalenylmethylether.

10. Verfahren nach Anspruch 1, wobei eine Verbindung der Formel **(II)** oder ein Salz davon durch ein Verfahren umfassend die Reaktion einer Verbindung der Formel **(IV)** 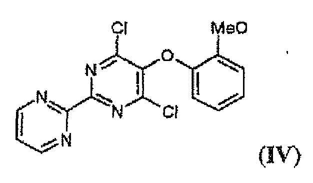 mit Ethylenglycol in der Anwesenheit einer Base erhalten wird.

11. Verfahren nach Anspruch 10, wobei die Base ausgewählt wird aus einem Tri-(C₁-C₆)Alkylamin, Diazabicyclooctan und Diazabicycloundecen oder Mischungen davon.

12. Verfahren nach Anspruch 10, wobei die Reaktion in der Anwesenheit eines Überschusses an Ethylenglycol durchgeführt wird.

13. Verfahren nach Anspruch 10, wobei die Reaktion in der Anwesenheit eines organischen Lösungsmittels durchgeführt wird und das Molverhältnis von Ethylenglycol zu einer Verbindung der Formel **(IV)** ungefähr von 1 bis 5 reicht.

## Revendications

1. Procédé pour la préparation de 4-tert-butyl-N-[6-(2-hydroxy-éthoxy)-5-(2-méthoxy-phénoxy)-[2,2']bipyrimidinyl-4-yl]-benzènesulfonamide, de formule (I), ou d'un sel ou forme hydratée de celui-ci, 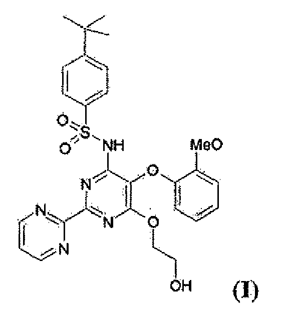 comprenant la réaction d'un composé de formule (II), ou d'un sel de celui-ci, 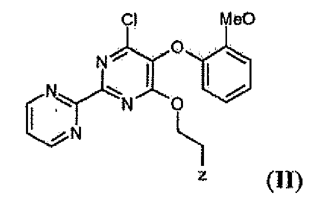 où Z est un groupe hydroxy éventuellement protégé, avec un composé de formule (III) ou un sel de celui-ci, 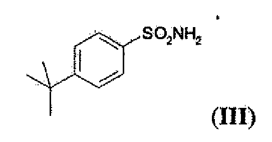 en présence d'une base; et, si nécessaire, l'élimination du groupe de protection hydroxy, et/ou, si désiré, la conversion d'un composé de formule (I) en un sel de celui-ci, ou *vice versa.*

2. Procédé selon la revendication 1, dans lequel le groupe de protection est choisi parmi un groupe acyle ; un groupe aryl C₁-C₆ alkyle; et un groupe tri (C₁-C₆) alkyl-silyle.

3. Procédé selon la revendication 1, dans lequel la base est choisie parmi un C₁-C₆ alcoolate de métal alcalin; un hydroxyde, carbonate ou phosphate de métal alcalin ou alcalino-terreux.

4. Procédé selon la revendication 2, dans lequel la base est un phosphate de métal alcalin ou alcalino terreux.

5. Procédé selon la revendication 1, dans lequel le rapport molaire d'un composé de formule (III) par rapport à un composé de formule (II) est dans un domaine approximativement de 1 à 2.

6. Procédé selon la revendication 1, dans lequel le rapport molaire de la base par rapport à un composé de formule (II) est dans un domaine approximativement de 1 à 5.

7. Procédé selon la revendication 1, dans lequel la réaction est réalisée en présence d'un catalyseur, et, si nécessaire d'un ligand.

8. Procédé de formule (II) ou un sel de celui-ci, 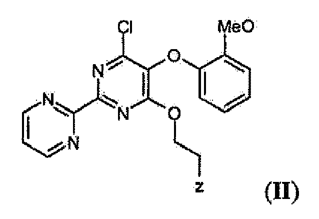 où Z est un groupe hydroxy protégé.

9. Composé de formule (II) selon la revendication 8, qui est :
2-(5-(2-méthoxy-phénoxy)-6-chloro-2-(pyrimidin-2-yl)pyrimidin-4-yloxy) éthanol formyl ester;
2-(5-(2-méthoxy-phénoxy)-6-chloro-2-(pyrimidin-2-yl)pyrimidin-4-yloxy) éthanol acétyl ester;
2-(5-(2-méthoxy-phénoxy)-6-chloro-2-(pyrimidin-2-yl)pyrimidin-4-yloxy) éthanol benzoyl ester;
2-(5-(2-méthoxy-phénoxy)-6-chloro-2-(pyrimidin-2-yl)pyrimidin-4-yloxy) éthanol triméthylsilyl éther;
2-(5-(2-méthoxy-phénoxy)-6-chloro-2-(pyrimidin-2-yl)pyrimidin-4-yloxy) éthanol tert-butyl-diméthylsilyl éther;
2-(5-(2-méthoxy-phénoxy)-6-chloro-2-(pyrimidin-2-yl)pyrimidin-4-yloxy) éthanol benzyl éther;
2-(5-(2-méthoxy-phénoxy)-6-chloro-2-(pyrimidin-2-yl)pyrimidin-4-yloxy) éthanol phényléthyl éther; ou
2-(5-(2-méthoxy-phénoxy)-6-chloro-2-(pyrimidin-2-yl)pyrimidin-4-yloxy) éthanol naphthalénylméthyl éther.

10. Procédé selon la revendication 1, dans lequel un composé de formule (II), ou un sel de celui-ci, est obtenu par un procédé comprenant la réaction d'un composé de formule (IV) 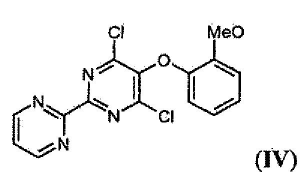 avec de l'éthylène glycol, en présence d'une base.

11. Procédé selon la revendication 10, dans lequel la base est choisie parmi une tri(C₁-C₆)alkylamine, un diazabicyclooctane et un diazabicycloundécène ou des mélanges de ces derniers.

12. Procédé selon la revendication 10, dans lequel la réaction est réalisée en présence d'un excès d'éthylène glycol.

13. Procédé selon la revendication 10, dans lequel la réaction est réalisée en présence d'un solvant organique, et le rapport molaire de l'éthylène glycol par rapport à un composé de formule (IV) est dans un domaine approximativement de 1 à 5.
